Europäisches Patentamt

European Patent Office

Office européen des brevets

(19)

(11) Publication number: **0 342 280**

**A1**

(12) **EUROPEAN PATENT APPLICATION**

(21) Application number: 88304485.1

(51) Int. Cl.⁴: **C12P 19/18**

(22) Date of filing: 18.05.88

(43) Date of publication of application:
23.11.89 Bulletin 89/47

(84) Designated Contracting States:
**BE DE ES FR GB IT NL SE**

(71) Applicant: **UOP INC.**
**25 East Algonquin Road**
**Des Plaines Illinois 60017-5017(US)**

(72) Inventor: **Rohrbach, Ronald P.**
**21 W 755 Ravine Road**
**Forest Lake Illinois 60047(US)**
Inventor: **Scherl, Dale S.**
**1304 Sir Lancelot Drive**
**Mount Prospect Illinois 60056(US)**

(74) Representative: **Brock, Peter William**
**URQUHART-DYKES & LORD 91 Wimpole**
**Street**
**London W1M 8AH(GB)**

(54) Hydrolysis of starch to cyclodextrins.

(57) The yield of cyclodextrins formed in the hydrolysis of starch catalyzed by a cyclodextrin glycosyltransferase can be substantially increased by the addition of certain water-soluble solutes. Alcohols having 1 to 6 carbon atoms form one such class, with ethanol and isopropyl alcohol being especially effective. Another class of solute comprises salts with complexing anions (halides, perchlorates, thiocyanates, and oxoanions of pentavalent phosphorus). When an immobilized cyclodextrin glycosyltransferase is used, yields of beta-cyclodextrin can be increased from approximately 20% to almost 40%, using 15% isopropyl alcohol in a thinned starch feedstock, with glucose·production being increased by only about 25%.

*Figure I*

ENHANCEMENT OF BETA-CD PRODUCTION WITH ISOPROPANOL

EP 0 342 280 A1

## HYDROLYSIS OF STARCH TO CYCLODEXTRINS

This invention relates to the hydrolysis of starch to cyclodextrins employing a cyclodextrin-enhancing solute.

Cyclodextrins are cyclic molecules consisting of 1.4 linked alpha-D-glucopyranose monomer units. The cyclodextrins containing 6-, 7-, and 8-membered rings, commonly known as alpha-, beta-, and gamma-cyclodextrin, respectively, are the most important cyclodextrins to date, possibly because of their availability relative to cyclodextrins of different ring size. Th usefulness of these cyclodextrins arises from their ability reversibly to form inclusion complexes (clathrates) with many types of compounds. Inclusion complexes arise when a host molecule, such as a cyclodextrin, has a structure containing an interior cavity into which guest molecules can bind by weak interactions such as van der Waal's forces. The latter are short range forces which are sufficiently strong to allow the formation of definite (generally solid) complexes, but are sufficiently weak to permit ready dissociation of the complex into a host and guest molecule.

The cyclodextrins are doughnut-shaped molecules with an interior cavity whose size and shape is determined by the number of glucose units that make up the ring. In alpha-cyclodextrin the almost cyclindrical cavity is approximately 7 Angstroms (0.7 nm) deep and 5 Angstroms (0.5 nm) in diameter. In beta-cyclodextrin the depth is the same, but the diameter is 7 Angstroms (0.7 nm), while in gamma-cyclodextrin the cavity is again 7 Angstroms (0.7 nm) deep but is 9 Angstroms (0.9 nm) in diameter. Cyclodextrins are soluble in water because of the many hydroxyl groups of the glucose subunits that surround the rim of the cavity. The interiors of the cavities themselves, however are hydrophobic, and these hydrophobic cavities extract organic molecules from aqueous solution if the organic materials have the correct shape and hydrophobic character.

The complexing ability of cyclodextrins lends itself to various uses. For example, the cyclodextrins are used in encapsulating desirable flavours and fragrances, which can then be stored for reasonably long periods and added to foods during their preparation. Alternatively, cyclodextrins may be used in removing undesirable flavours and fragrances from food by complexing with them. Cyclodextrins also are used in the Protection of foods against oxidation, photochemical degradation, and thermal decomposition. These and other uses have been summarised by J. Szejtli, **STARCH, 34,** 379-385 (1982).

To date, cyclodextrins have been prepared by treating starch with a cyclodextrin glycosyltransferase (CG), initially at a high temperature to liquefy the starch, and then at a lower temperature to form the . cyclodextrins from the liquefied starch. Although many variations are possible, all utilise a liquid starch of low dextrose equivalent (DE), less than 4, as a substrate for the enzyme. The prior art methods have been described and summarised by K. Horikoshi, **PROCESS BIOCHEMISTRY,** May, 1979, 26-30, and by M. Matzuzawa et al., **DIE STARKE, 27,** 410-413 (1975).

The use of thinned starch, that is, a partially hydrolised starch, as a feedstock, can obviate some of the above-mentioned difficulties experienced in the case of an immobilised cyclodextrin glycosyltransferase (IMCG), but whether a soluble or immobilised cyclodextrin glycosyltransferase is used, the present methods of cyclodextrin manufacture suffer from several serious inherent limitations, which severely restrict cyclodextrin manufacture. In both instances the solids content of the feedstock is relatively low, which limits cyclodextrin productivity, i.e., the amount of cyclodextrin formed per unit time. The total cyclodextrin yield also is relatively low, with total conversions of starch below 50 percent, often in the range of 25-35 percent. The low cyclodextrin yield merely exacerbates the low productivity associated with the low feedstock solids content. The low solids content of the feedstock necessary for present methods of cyclodextrin manufacture also results in increased processing costs downstream, for the cyclodextrins will necessarily be formed in a relatively dilute solution, which requires the removal of relatively large amounts of water in a cyclodextrin purification stage. Since the removal of water from the product is an energy-intensive operation, this translates directly into increased production and costs.

Bacterial contamination of polysaccharide feedstocks for enzyme-mediated processes is a common problem amenable to several convenient solutions. During the evaluation of one such solution, it was found that the addition of ethanol to a starch feedstock as a bacterial inhibitor also caused, quite unexpectedly, a significant increase in cyclodextrin yield. A subsequent focused investigation then showed that the presence of certain water-soluble materials in the feedstock had a beneficial effect on cyclodextrin yield, and in some instances, that effect was profound. Still further investigation demonstrated that such added solutes not only increased cyclodextrin yield, but also permitted the use of feedstocks with higher solids content, thereby increasing productivity and reducing processing costs. Thus, it has now been found that the addition of some water-soluble materials to a starch feedstock simultaneously prevents bacterial contamination, increases cyclodextrin yield, increases cyclodextrin Productivity, and reduces cyclodextrin processing costs.

The purpose of the invention described and claimed herein is to increase the yield and productivity of cyclodextrin formed by hydrolysis of starch catalyzed by cyclodextrin glycosyltransferase. The broadest embodiment comprises performing the hydrolysis in the presence of an effective amount of a cyclodextrin-enhancing water-soluble solute. In a more specific embodiment, the solute is an organic alcohol having up to 6 carbon atoms. In a still more specific embodiment, the alcohol is ethanol or isopropyl alcohol in an amount from 5 to 40% by volume. Alternatively, the solute can be a salt having a halide, perchlorate or thiocyanate anion, or an oxoanion of pentavalent phosphorus. Other specific embodiments will be apparent from the following description.

The invention will be illustrated with reference to the accompanying Drawings, in which:

Figure 1 shows the production of beta-cyclodextrin with time in the absence and presence of 15 volume percent of isopropyl alcohol.

Figure 2 shows the production of glucose with time in the absence and presence of 15 volume percent of isopropyl alcohol.

Figure 3 shows the time course of beta-cyclodextrin production in the presence of 7.5 volume percent ethanol or isopropyl alcohol.

As a class of materials, cyclodextrins have intriguing properties making them attractive for various commercial uses, as has been described above. The production of cyclodextrin is, however, plagued by low yields and low productivity. This invention increases both starch conversion to cyclodextrins and the amount of cyclodextrins formed per unit time using cyclodextrin glycosyltransferase to catalyze starch hydrolysis, and especially when using an immobilised cyclodextrin glycosyltransferase.

The feedstocks that are used for cyclodextrin production, and which are used in the practice of this invention, are aqueous starch solutions or suspensions, generally containing small amounts of other materials such as buffers, preservatives, etc. The feedstock may be raw starch, a liquified starch, or a thinned starch whose dextrose equivalent (D.E.) may be as low as 0 and as high as 40, although a feedstock with dextrose equivalent between 2 and 25 is preferred. The use of thinned starch is particularly favoured when an immobilised cyclodextrin glycosyltransferase is used for starch hydrolysis, whereas traditionally this is not used with the soluble enzymes.

It is preferred that the starch feedstock should have as high a solids content as possible, to maximise the productivity of cyclodextrin production. However, one of the hydrolysis products is glucose, a known glycosyltransferase inhibitor, a fact which in the past has limited the dry solids content to a level below 10%. In the practice of the present invention, a feedstock with a dry solids content between 1 and 30% of even higher, may be used, with those having a dry solids content between 2 and 20% being particularly preferred.

The cyclodextrin glycosyltransferase which is used to convert the starch into cyclodextrins, may be used either as a soluble enzyme or as an immobilised enzyme. When an immobilised enzyme is used, the success of the invention does not depend upon the nature of the support matrix; it is intended that any support matrix may be used to immobilise cyclodextrin glycosyltransferase (IMCG) in the practice of the present invention, and in this sense the IMCG referred to herein is a generic designation. This is not to say that the nature of the support matrix is immaterial, but merely that any preference for a particular support matrix arises from factors incidental to and unconnected with this invention.

The results attending the addition of the solutes used according to invention depend upon the enzyme source. It appears that there are at least two classes of cyclodextrin glycosyltransferase. One class, as exemplified by the enzyme produced by **B. circulans**, can be characterised as a beta- and a low alpha-producer, for the mixture of cyclodextrins formed comprises approximately 5% alpha-, 20% beta-, and 2% gamma-cyclodextrin. On the other hand, a second class of enzymes, as exemplified by that produced from **B. macerans**, can be characterised as a high beta- and alpha- producing enzyme, for the cyclodextrins formed, comprise approximately 17% alpha-, 17% beta-, and 5% gamma-cyclodextrin. In general, for the cyclodextrin glycosyltransferases of the first type, the solutes of the invention tend dramatically to increase the beta-cyclodextrin, decrease the alpha-cyclodextrin, and cause a smaller increase in the gamma-cyclodextrin. In contrast, where a high alpha-producing enzyme is used, the solutes of the invention primarily increase the yield of the alpha-cyclodextrin with little, or perhaps even a small negative, effect on beta-cyclodextrin production. It is clear then that the specific effect caused by the solutes of the invention will depend upon the particular enzyme used, but in all instances the total yield of cyclodextrins will be increased.

The present invention is based on the discovery that the addition of certain water-soluble materials to starch feedstocks being hydrolyzed by cyclodextrin glycosyltransferase, increases the yield of cyclodextrins

3

over that formed in the absence of these materials. The solutes which are effective may be either organic solutes or salts. Among the favoured organic solutes are alcohols containing from 1 to 6 carbon atoms, such as methanol, ethanol, and the isomers of propanol, butanol, pentanol, and hexanol. Alcohols containing 2, 3, or 4 carbon atoms are preferred, and both ethanol and isopropyl alcohol are highly preferred solutes in the practice of this invention. One reason why isopropyl alcohol is so highly preferred, when used with certain cyclodextrin glycosyltransferases, is that it substantially increases the yield of beta-cyclodextrin with a much smaller attendant increase in glucose formation. As was previously mentioned, glucose is an enzyme inhibitor, so that its formation is desirably minimised.

Other water soluble organic materials alternatively can be used, including such lower ketones as ethyl ketone and methyl ethyl ketone. The class of organic solutes which may be used in the practice of this invention can be phenomenologically and operationally defined. Firstly, they need to be soluble, at least to the extent of 5% at 50° C, on a volume-volume basis when the solute is a liquid, and on a weight-volume basis where the solute is solid. Secondly, they must complex with one or more of the cyclodextrins, but the complex must be soluble in the reaction mixture under reaction conditions. The solute also should have no detrimental effect on the stability of the enzyme, as evidenced by its half-life, or otherwise adversely affect enzyme performance. It is also important that the solute should be readily seperable from the cyclodextrins which are subsequently isolated, and it is preferable that the solutes themselves should be approved for food use, at least at the low levels at which they may be expected to accompany the isolated cyclodextrin.

As previously stated, salts may alternatively be used in the practice of this invention, so long as they conform to the phenomenological and operational definitions stated above. It has been found that the effect of salt solutes is virtually independent of the cation in the salt, which implies that the cyclodextrin-enhancing properties arise only from the anion. This is consistent with the known complexing tendencies of cyclodextrins. Among the anions which may be used are included the halides, especially iodide, bromide, and to a lesser extent chloride, perchlorate, thiocyanate, and the oxoanions of pentavalent phosphorus, such as phosphate, hydrogen phosphate, dihydrogen phosphate, pyrophosphate, etc. When salts are used in the practice of this invention, they may be employed at a concentration of 0.1 to 2M. When organic solutes are used, it has been found that they are effective at a concentration between 5 and 40%, although normally their concentration does not exceed 30%, where percentages are volume-volume or weight-volume, depending respectively upon whether the solute is liquid or solid.

Other than the presence of the solutes of this invention, there is no difference in the conditions under which starch hydrolysis to cyclodextrins is conducted. Thus, hydrolysis generally is performed at a temperature between 45 and 70° C, even more usually between 50 and 60° C. The starch feedstock generally is buffered, with the hydrolysis generally being performed at a pH between 5.5 and 7.5. In any event, the conditions for hydrolysis of starch into cyclodextrins as catalyzed by cyclodextrin glycosyltransferase, are well known to those in the art and need not be further discussed here.

The Examples which follow are only illustrative of this invention and do not limit it in any significant way. Unless otherwise stated, the cyclodextrin glycosyltransferase was obtained from **Bacillus circulans**. Its immobilization typically was carried out in the following way.

To 1.0 g of beta-alumina (60/80 U.S. mesh) (0.177 to 0.250 mm) was added 10.0 ml of a 1.8% aqueous solution of polyethyleneimine (PEI) at pH 10.5. Impregnation of the agitated mixture was continued for 1 hour under vacuum at room temperature and the PEI-coated support was thereafter separated by filtration, and then allowed to air-dry at room temperature for 2 days. To this PEI-coated support was added 10.0 ml of a 5.0% aqueous solution of glutaraldehyde. Excess liquid was removed by decantation, and the solid was washed copiously with deionized water to remove excess glutaraldehyde. Washing was continued until the filtrate gave a negative Fuchsin test, after which it was washed 3 or 4 times with a 1 millimolar sodium acetate buffer at pH 5.0.

A solution of purified cyclodextrin glycosyltransferase was adjusted to pH 5.0 by addition of 1.0 molar sodium acetate. To the activated support prepared as described above, was added the solution of glycosyltransferase, and the mixture was shaken on an orbital shaker at 4° C overnight with a cycle of 15 seconds on and 5 minutes off. Excess liquid was then removed by decantation and the immobilised enzyme preparation was washed well, in order to remove adhering but unbound enzyme. The resulting immobilised cyclodextrin glycosyltransferase is hereinafter referred to as IMCG.

Soluble cyclodextrin glycosyltransferase was assayed using a substrate of 0.2% soluble starch at pH 7.0 containing calcium chloride (5 millimolar) and imidazole (100 millimolar). To 0.30 ml of the substrate maintained in a 40° C water bath was added 10 microliters of an enzyme solution. After 10 minutes, the reaction is quenched with 4.0 ml of 0.2 molar hydrochloric acid, and there is added 0.5 ml of a solution containing 0.02% of iodine and 0.20% of potassium iodide. At the same time, a control is prepared using 0.30 ml of the substrate to which is added 4.0 ml of 0.2 molar hydrochloric acid, followed by 10 microliters

4

of the enzyme solution whose activity is being assayed, and 0.5 ml of the I$_2$-KI solution. The absorbance at 700 nanometers was then measured on both the control and the sample, with activity calculated according to the equation,

$$\text{Activity (units/ml)} = \frac{[\text{OD(control)} - \text{OD(sample)}] \times 100}{\text{OD(control)} \times 0.01}$$

The difference in absorbance between the control and sample must be between 0.1 and 0.4 absorbance units. If values are not within this range, the enzyme solution is diluted, and such dilutions are then factored into the calculation.

## EXAMPLE 1

Effect of ethanol.

In an effort to reduce microbial contamination, 4% by volume of ethanol was added to partially hydrolyzed starch, D.E.15, 2% dry solids, which also contained calcium chloride (5 mM) and imidazole (5 mM), which was then used as the feedstock for an IMCG at 50°C. In addition to the feedstock being contamination-free over a period of 19 days, the amount of beta-cyclodextrin formed was increased from 15 to 25%. There also was observed no significant change in the stability of the catalyst after 19 days.

As a result of the preceding observation, a scan of the effect of ethanol on the formation of cyclodextrins was performed in the following way. The feedstock described above, but containing various amounts of ethanol, was used as the feedstock for soluble cyclodextrin glycosyltransferase. The amount of each cyclodextrin was determined by high pressure liquid chromatography (HPLC), under conditions where there was complete resolution of the alpha-, beta-, and gamma-cyclodextrins. The following Table 1 summarises the results obtained.

Table 1

| Effect of Ethanol Concentration on Cyclodextrin Production | | | |
|---|---|---|---|
| % Ethanol[a] | Alpha[b] | Beta[b] | Gamma[b] |
| 0 | 0.156 | 0.321 | 0.009 |
| 5 | 0.165 | 0.392 | 0.012 |
| 10 | 0.133 | 0.474 | 0.029 |
| 15 | 0.096 | 0.527 | 0.031 |
| 20 | 0.074 | 0.572 | 0.032 |
| 25 | 0.048 | 0.572 | 0.045 |
| 40 | 0.030 | 0.647 | 0.060 |

a. Volume percent
b. Concentration in weight percent.

As these data show, the amount of beta-cyclodextrin formed increases appreciably with the amount of ethanol added. The most significant changes appear to occur in the range up to 25 volume percent of ethanol.

## EXAMPLE 2

Effect of Organic Solutes.

The feedstocks used were a 2.0 weight percent solution of partially hydrolyzed starch, D.E. 15, at pH 7.0, 5 mM in both CaCl₂ and imidazole, and containing varying amounts of organic solutes. To 1.0 ml of feedstock was added 10 microliters of an enzyme solution, and the reaction at 40° C was continued for 1 hour. The solution was quenched by heating to boiling and was then analyzed by HPLC as described above. Results are summarised in the following Table 2.

Table 2

| Effect of Some Organic Solutes on Beta-cyclodextrin formation | | |
|---|---|---|
| Solute | Concentration[a] | Beta-cyclodextrin[b] |
| None | | 18.6 |
| ethanol | 5 | 23.0 |
| 2-propanol | 5 | 29.1 |
| isobutyl alcohol | 5 | 28.8 |
| sec-butyl alcohol | 5 | 32.1 |
| tert-butyl alcohol | 5 | 35.8 |
| none | | 18.7 |
| methanol | 20 | 21.0 |
| ethanol | 20 | 24.4 |
| 2-propanol | 20 | 25.6 |
| acetone | 20 | 30.2 |

a. Concentration is in volume-volume percent
b. Concentration is in weight percent relative to starch conversion.

These results show that the lower alcohols, as a class, are effective in increasing the yield of beta-cyclodextrin, but that the effects of the alcohols depend both upon the nature of the alcohol and upon its concentration. The use of tert-butyl alcohol as a solute, at a concentration of 5 volume percent, approximately doubles the yield of beta-cyclodextrin.

**EXAMPLE 3**

Effect of isopropyl alcohol on beta-cyclodextrin and glucose production using IMCG.

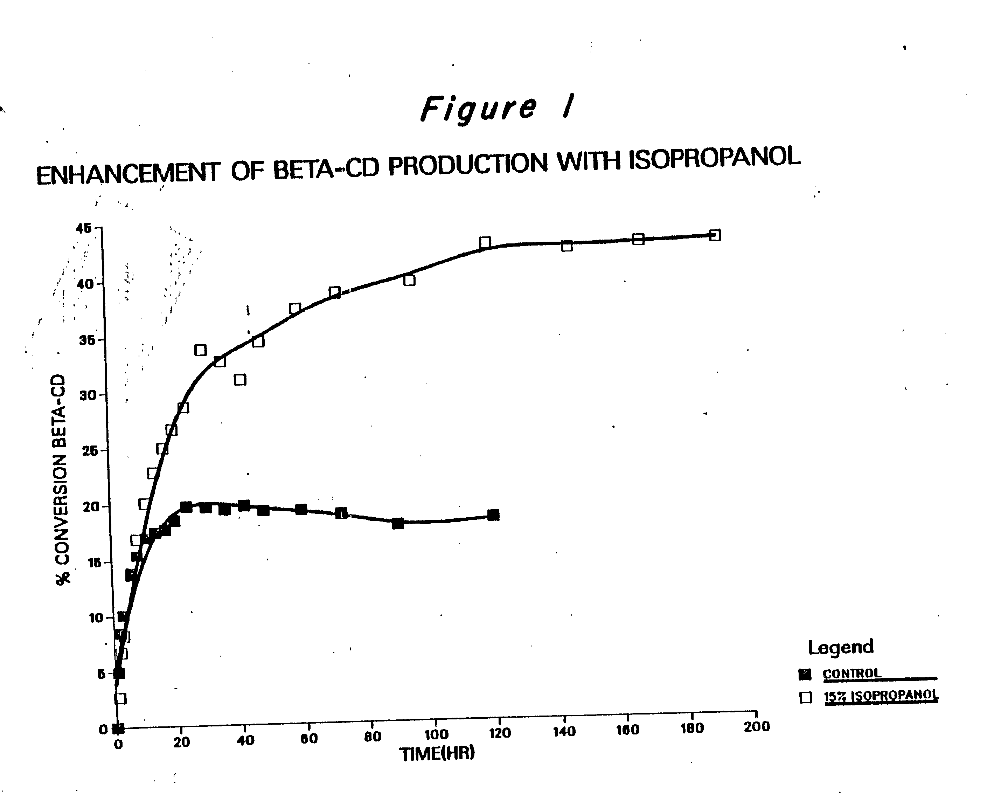

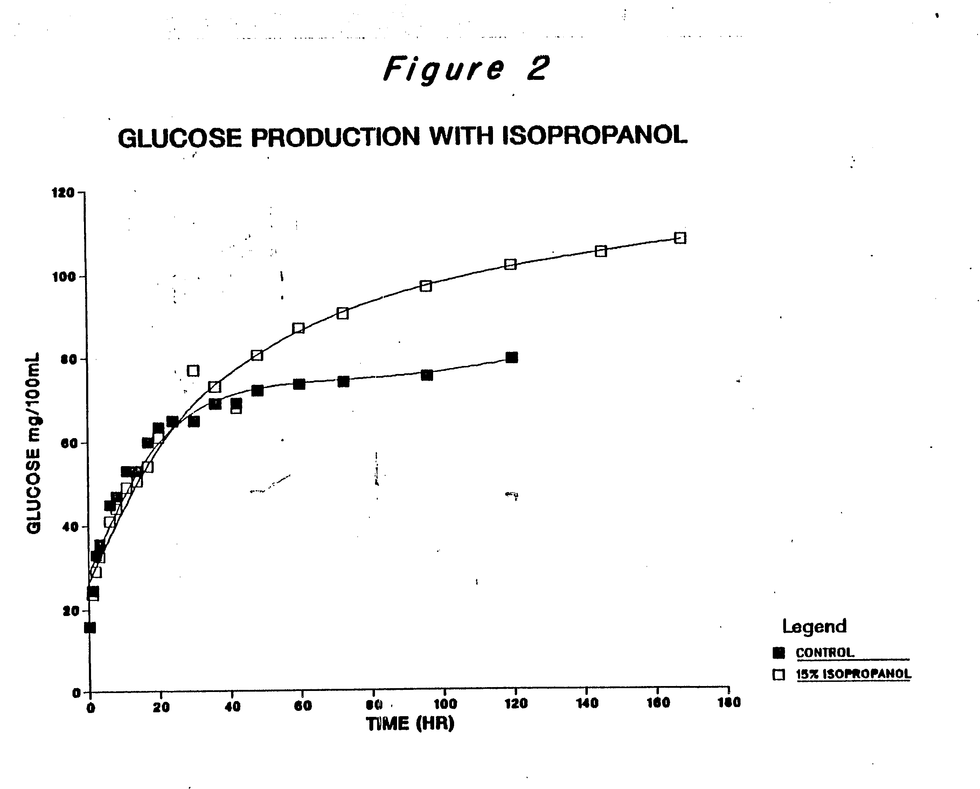

A feedstock of 2 weight percent of partially hydrolyzed starch of D.E. 15 (Maltrin 150) containing 15 volume percent of isopropyl alcohol, and 5 millimolar in both calcium chloride and imidazole, buffered to pH 7.0, was used in a batch recycle mode with IMCG. Figures 1 and 2 of the accompanying Drawings show the time course of both beta-cyclodextrin and glucose formation. These data show that, relative to the control, addition of isopropyl alcohol at 15 volume percent roughly doubles the yield of cyclodextrin. It is also important to note that the glucose yield is increased in the order of only about 30%. Since glucose is an inhibitor for cyclodextrin glycosyltransferase, this lower increase in glucose production, relative to beta-cyclodextrin formation, means that the latter gain is accompanied with only a small inhibitory "penalty".

**EXAMPLE 4**

Effect of ethanol and isopropyl alcohol on beta-cyclodextrin formation using IMCG.

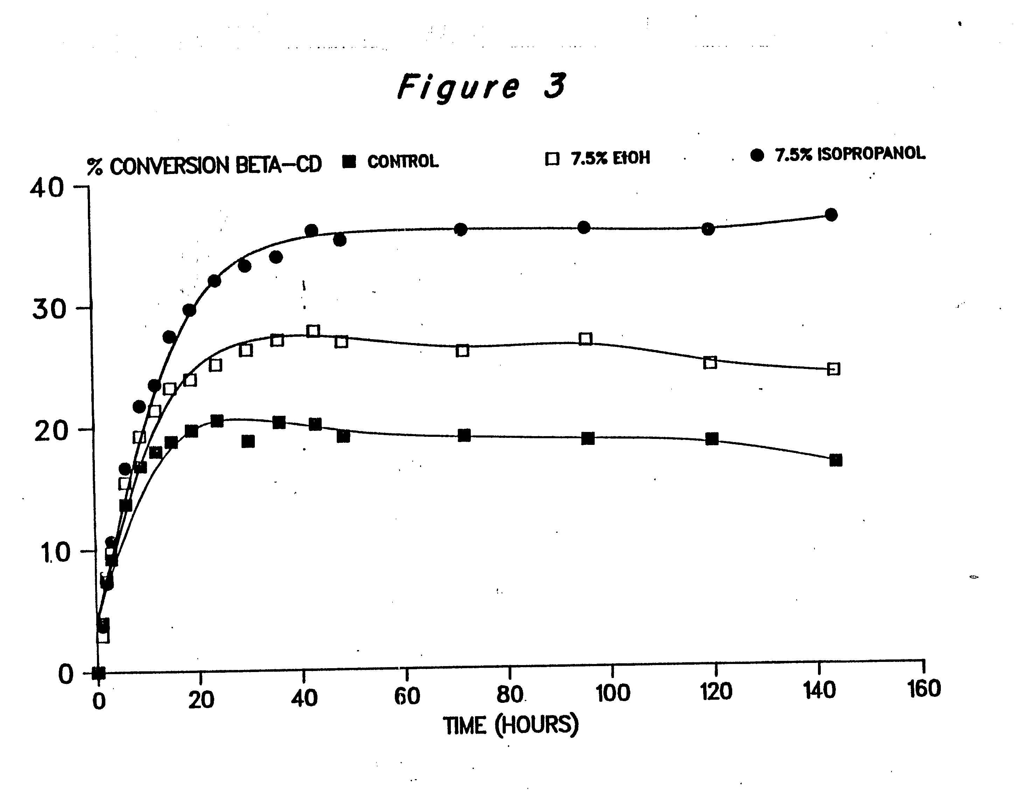

Experiments similar to that described in the previous Example were performed using 7.5 volume percent of ethanol or isopropyl alcohol as the added solute. The time course of maximum beta-cyclodextrin production is shown in Figure 3 of the accompanying Drawing, which shows that the presence of ethanol increases beta-cyclodextrin production from 19 to 25%, whereas 7.5 volume percent of isopropyl alcohol raises it to 36%.

## EXAMPLE 5

Effect of ionic strength on beta-cyclodextrin formation.

The effect of various concentrations of sodium sulphate on beta-cyclodextrin formation, using soluble enzyme and a feedstock of 2% of a partially hydrolyzed starch of D.E. 15, at a pH of 7.0, was determined, with the results summarised in the following Table 3.

Table 3

| Salt Concentration and Beta-cyclodextrin Formation | |
| --- | --- |
| $Na_2SO_4$ Molarity | Percent Beta-cyclodextrin Yield |
| 0 | 18.0 |
| 0.1 | 17.8 |
| 0.2 | 17.6 |
| 0.3 | 16.7 |
| 0.4 | 16.1 |
| 0.5 | 15.7 |

These results show that ionic strength, resulting from a noncomplexing anion, has a negative effect on beta-cyclodextrin formation.

## EXAMPLE 6

Effect of certain anions on beta-cyclodextrin formation.

The effect of various inorganic anions on beta-cyclodextrin yield was determined as described in the previous Example, using the specified salts added in the amount indicated. Results are summarised in the accompanying Table 4, which shows an increase in beta-cyclodextrin formation relative to that formed in the presence of sodium sulphate alone.

Table 4

| Comparative Effect of Halide Salts | |
|---|---|
| Na$_2$SO$_4$ Molarity | Percent Beta-cyclodextrin yield |
| Na$_2$SO$_4$, 1.0M<br>KCl, 1.0M<br>KI, 1.0M | 17.3<br>14.0<br>17.4<br>20.9 |

Claims

1. A method of making cyclodextrins by hydrolyzing a starch feedstock in the presence of a cyclodextrin glycosyltransferase, characterized in that hydrolysis is carried out in the presence of a cyclodextrin-enhancing water-soluble solute.

2. A method according to claim 1 characterized in that the solute is an organic solute having a concentration from 5 to 40 percent.

3. A method according to claim 2 characterized in that the solute is an alcohol having up to 6 carbon atoms.

4. A method according to claim 3 characterized in that the alcohol is present in an amount from 5 to 30% by volume.

5. A method according to claim 3 or 4 characterized in that the alcohol is ethanol or an isomer of propanol or butanol.

6. A method according to claim 1 characterized in that the solute is a salt having a halide, perchlorate or thiocyanate, anion or an oxoanion of pentavalent phosphorus.

7. A method according to claim 1 characterized in that the feedstock is a partially hydrolyzed starch of dextrose equivalent from 2 to 25 containing from 5 to 30% by volume of ethanol or isopropyl alcohol and the hydrolysis is performed by an immobilized cyclodextrin glycosyltransferase.

8. A method according to any one of claim 1 to 7 characterized in that the solute is added in an amount sufficient to increase the yield of beta-cyclodextrin by at least 30%.

# Figure 1

## ENHANCEMENT OF BETA-CD PRODUCTION WITH ISOPROPANOL

EP 0 342 280 A1

# Figure 2

## GLUCOSE PRODUCTION WITH ISOPROPANOL

**Legend**

■ CONTROL

□ 15% ISOPROPANOL

## Figure 3

% CONVERSION BETA—CD   ■ CONTROL      □ 7.5% EtOH      ● 7.5% ISOPROPANOL

TIME (HOURS)

EP 0 342 280 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, vol. 5, no. 31 (C-45)[703], 25th February 1981; & JP-A-55 156 595 (TOYO JOZO K.K.) 05-12-1980 * Abstract * | 1-5,8 | C 12 P 19/18 |
| X | PATENT ABSTRACTS OF JAPAN, vol. 11, no. 8 (C-396)[2455], 9th January 1987; & JP-A-61 185 196 (SHOKUHIN SANGYO BAIORIAKUTAA SYST GIJUTSU KENKYU KUMIAI) 18-08-1986 * Abstract * | 1-5,8 . | |
| Y | CHEMICAL ABSTRACTS, vol. 96, no. 23, 7th June 1982, page 710, abstract no. 200045n, Columbus, Ohio, US; H. HOEILAND et al.: "Volume change on complex formation between anions and cyclodextrins in aqueous solution", & J. SOLUTION CHEM. 1981, 10(11), 775-84 * Abstract * | 1-8 | |
| Y | US-A-3 640 847 (ARMBRUSTER et al.) * Claims * | 1-8 | TECHNICAL FIELDS SEARCHED (Int. Cl.4) C 12 P C 08 B |
| Y | US-A-3 652 398 (ARMBRUSTER et al.) * Claims * | 1-8 | |

The present search report has been drawn up for all claims

| Place of search | Date of completion of the search | Examiner |
|---|---|---|
| THE HAGUE | 24-01-1989 | LENSEN H.W.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)